# EUROPEAN PATENT SPECIFICATION

(11) **EP 1 436 407 B1**
(45) Date of publication and mention of the grant of the patent: **06.12.2006**
(21) Application number: 02762559.9
(22) Date of filing: 18.09.2002
(51) Int. Cl.: C12Q 1/00, G01N 27/327

(54) **AMPEROMETRIC SENSOR**
AMPEROMETRISCHER SENSOR
CAPTEUR AMPEROMETRIQUE

(30) Priority: 18.10.2001 GB 0125094
(43) Date of publication of application: 14.07.2004
(73) Proprietor: DREW SCIENTIFIC CO. LIMITED, Dallas, Texas 75237-1023 (US)
(72) Inventor: SLATER, Jonathan, M., Birbeck Univ. of London, Bloomsbury, London WC1H 0P (GB); MURPHY, Lindy, Birbeck Univ. of London, Bloomsbury, London WC1H 0P (GB); LAU, Kim, National Centre for Sensor Research, Glasnevin, Dublin 9 (IE)
(74) Representative: McCluskie, Gail Wilson
(86) International application number: PCT/GB2002/004268
(87) International publication number: WO 2003/033726

(56) References cited:
- WO-A-00/20626
- WO-A-92/07263
- US-A- 4 711 245
- US-A- 5 288 636
- US-A- 5 385 846

## Description

In general terms the present invention relates to the determination of the concentration of an analyte in a sample. More specifically, the invention relates to an amperometric sensor, to its use, to cartridges for the sensor and to redox mediator compounds for use in the sensor.

A number of electrochemical sensors (or biosensors) have been proposed previously. For example, US 5,288,636 describes a sensor useful for determining glucose concentration in a sample and relies on the reaction between the enzyme glucose oxidase and glucose with the mediator potassium ferricyanide to produce a ferrocyanide which is then electro-oxidised to produce a measurable current that is representative of the concentration of glucose present.

The reactions involved can be summarised as follows:
1. GOD_{OX} + glucose → GOD_{RED} + gluconolactone
2. GOD_{RED} + M_{OX} → GOD_{OX} + M_{RED}
3. M_{RED} → M_{OX} + e⁻ [Signal]
   GOD_{OX}- oxidised form of glucose oxidase
   GOD_{RED} - reduced form of glucose oxidase
   M_{OX} - oxidised form of mediator (ferricyanide)
   M_{RED} - reduced form of mediator (ferrocyanide)

In step 1 the enzyme oxidises the glucose and is itself reduced. In step 2 the reduced form of the enzyme reacts with the oxidised form of the mediator to produce the reduced form of the mediator and to regenerate the oxidized form of the enzyme. In step 3 the oxidised form of the mediator is regenerated by electro-oxidation. A measurable current/signal is generated. Thus, this type of sensor depends on reaction between the mediator and enzyme.

The use of a quinone-hydroquinone mediator system with glucose oxidase is known (Stoytcheva et al, Analytica Chimica Acta, 315 (1995) pp 101-107). However, unsubstituted benzoquinone has the disadvantage of having a relatively high oxidation potential of approximately +400 mV (versus SCE).

US 4,711,245 also describes a sensor for determining glucose concentration. The sensor relies on a reaction involving glucose, the enzyme glucose oxidase and the oxidised form of a substituted ferrocene. The ferrocene is reduced and then reoxidised to produce an easily measurable current.

WO 92/07263 provides a biochemical process involving electron transfer between a redox system and an electrode comprising a paste of electrically conductive particles and a pasting material and a charge transfer mediator, wherein the mediator is a compound of formula (X) 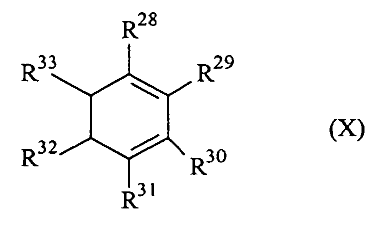 wherein R²⁸ and R³¹ are the same or different and each represent amino, alkylamino, diaalkylamino, morpholinyl or piperidyl, and R²⁹, R³⁰ and R³³ are the same or different and each represents hydrogen or alkyl, preferably hydrogen, R³² represents hydrogen, alkyl or nitro, preferably hydrogen or nitro, or R²⁸ together with R³³ either represents a moiety of the formula Xa 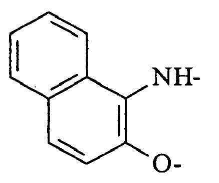 wherein the amino group is in the R²⁸ end, or R²⁸ together with R³³ represents a moiety of the formula Xb 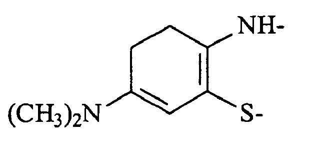 wherein the amino group is in the R²⁸ end.

WO 00/20626 provides a sensor for determining the concentration of an analyte in a sample fluid, where the sensor uses a non-leachable or a diffusible redox mediator. The sensors may be used in amperometry.

US 5,385,846 provides a biosensor and method for determining the haematocrit level of a whole blood sample. The biosensor comprises a first insulating substrate, working and counter electrodes, a second insulating substrate and a porous substrate which is impregnated with an electroactive compound.

There are various advantages associated with mediated sensors. Firstly, the kinetics for the oxidation of reduced enzyme can be faster with the mediator than with the natural electron acceptor oxygen. This can result in the sensor response being independent of oxygen tension. Secondly, in known sensors a potential is applied between electrodes in order to oxidize the reduced form of the mediator. The potentials sufficient to achieve this can be lower than for known sensors based on oxidation of hydrogen peroxide. This can result in a reduced oxidation of interferants present in the system, for example, ascorbate, urate and paracetamol.

The solubility of the mediator is an important factor in obtaining a measurable sensor response. The use of mediated sensors in flow systems can be limited by the solubility of the mediator. A readily soluble mediator can be lost rapidly to the carrier solution, resulting in severely reduced operating lifetime of the sensor. Alternatively, where the sensor is used in static solution and possibly with minimal sample volume, such as an electrochemical blood glucose stick, the use of a mediated sensor is enhanced by the solubility of the mediator. A poorly soluble mediator will limit the mediated enzyme reaction, resulting in poor sensitivity of the sensor.

The present invention provides mediators suitable for use in flowing streams by use of a sensor reliant on relatively insoluble mediator. The present invention also provides mediators suitable for use in static solution by use of a sensor reliant on relatively soluble mediator.

Accordingly, the present invention provides an amperometric sensor suitable for determining the concentration of analyte in a sample, said sensor comprising an oxidase enzyme and a substituted 1,4-benzoquinone compound which, in oxidized form, functions as a mediator selective for reduced enzyme and which has an oxidation potential lower than that of unsubstituted 1,4-benzoquinone.

The enzyme is an oxidase type enzyme. For example, in a sensor for determining the concentration of glucose in a sample, the enzyme may be glucose oxidase. The reaction between the enzyme and the analyte yields reduced glucose oxidase, and the concentration of the reduced glucose oxidase can be determined by using the sensor response and correlating this to a corresponding glucose concentration.

Other analytes which may be determined using the sensor of the present invention include cholesterol, pyruvate, bilirubin, alcohol, lactate, sarcosine, glycerol, creatinine, triglycerides and cholesterol; USP 5,288,636 gives details of some of the relevant enzymes. These analytes may be measured if one or more suitable enzyme(s) and mediator are included in the sensor. The sensors are constructed so that the final enzyme reaction results in the formation of reduced enzyme, which can be detected by the substituted benzoquinone mediator.

Herein the term "mediator" as used herein, means a compound which is capable of undergoing an electrochemical, reversible oxidation-reduction reaction.

The mediator used in the present invention is a substituted 1,4-benzoquinone compound which in oxidized form is selective for the redox site of reduced enzyme, i.e. which is reduced on reaction with reduced enzyme. The mediator has satisfactory solubility in water and common organic solvents, and has a lower redox potential than the corresponding unsubstituted benzoquinone. Examples of suitable compounds include those of the following formulae (I): 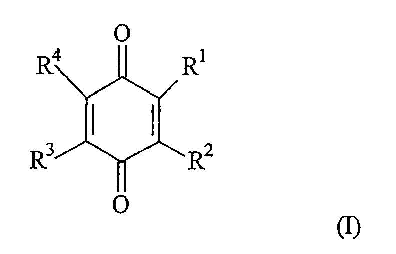 wherein R¹, R², R³ and R⁴ are the same or different and at least one of R¹, R², R³ and R⁴ is an alkyl group preferably C₁₋₃ alkyl or a phenyl group. Alternatively the groups R¹, R², R³ and R⁴ may be chosen from an alkoxyl group or a hydroxyalkyl group, preferably C₁₋₃ alkoxyl or hydroxy C₁₋₃ alkyl. Preferably the substituents are chosen from methyl, methoxy and hydroxymethyl groups.

Suitable 1,4-benzoquinone mediators for use in the sensor of the invention include:
methyl-1,4-benzoquinone
ethyl-1,4-benzoquinone
propyl-1,4-benzoquinone
2,5-dimethyl-1,4-benzoquinone
2,6-dimethyl-1,4-benzoquinone
trimethyl-1,4-benzoquinone
tetramethyl-1,4-benzoquinone
methoxy-1,4-benzoquinone
ethoxy-1,4-benzoquinone
propoxy-1,4-benzoquinone
2,5-dimethoxy-1,4-benzoquinone
2,6-dimethoxy-1,4-benzoquinone
trimethoxy-1,4-benzoquinone
tetramethoxy-1,4-benzoquinone
hydroxymethyl-1,4-benzoquinone
hydroxyethyl-1,4-benzoquinone
hydroxypropyl-1,4-benzoquinone
2,5-dihydroxymethyl-1,4-benzoquinone
2,6-dihydroxymethyl-1,4-benzoquinone
tri(hydroxymethyl)-1,4-benzoquinone
tetra(hydroxymethyl)-1,4-benzoquinone
phenyl-1,4-benzoquinone
2,3-dimethoxy-5-methyl-1,4-benzoquinone, and
2-hydroxymethyl-6-methoxy-1,4-benzoquinone.

An advantage of the substituted benzoquinones as defined herein is a reduction in oxidation potential compared to unsubstituted benzoquinone which has an oxidation potential of approximately + 400 mV (versus SCE). It has been shown that addition of methyl groups to 1,4-benzoquinone results in a negative shift in redox potential of approximately 55 mV per methyl group, due to the electron-donating nature of methyl groups (Driebergen et al., Analytica Chimica Acta, 233 (1990) pp 251-268). This enables measurements to be performed at lower oxidation potential and reduces the oxidation of interferent species present in samples such as ascorbate, urate and paracetamol. Examples of substituted 1,4-benzoquinones with lower oxidation potentials compared to 1,4-benzoquinone are shown in Table 1 below:

| Compound | Solubility (mg/L) | Eₒₓ (mV) |
|---|---|---|
| 1,4-benzoquinone | 1.11 E+04 (SRC) | 278 |
| 2,6-dimethyl-1,4-benzoquinone | 7.88 E+03 (SRC) | 137 |
| Tetramethyl-1,4-benzoquinone | 4.43 E+02(SRC) | -37 |
| Phenyl-1,4-benzoquinone | 1.14 E+03 (SRC) | 229 |
| 2,3-dimethoxy-5-methyl-1,4-benzoquinone | 1.11 E+04 (SRC) | 156 |
| 2,6-dimethoxy-1,4-benzoquinone | 7.06 E+04 (SRC) | 166 |
| 2-hydroxymethyl-6-methoxy-1,4-benzoquinone | 2.85E+05(C) | 158 |

SRC = values obtained from SRC PhysProp Database at http://esc.syrres.com/interlcow/
Eₒₓ = potential of oxidation peak obtained at a glassy carbon electrode,
using pH 6.95 bis-tris buffer and a Ag/AgCl/sat. KCl reference electrode.
C = calculated using KowWin from SRC PhysProp Database at http://esc.syrres.com

An additional advantage of use of the substituted benzoquinones is in the ability of these mediator compounds to oxidize reduced enzyme in place of the physiological electron acceptor, oxygen. This results in a sensor response that is independent of the oxygen tension of the sample. This is an advantage compared to sensors based on the measurement of hydrogen peroxide production or oxygen depletion caused by the reaction between enzyme and substrate.

Suitable benzoquinones for use in the sensor of the invention may be selected on the basis of their solubility compared to benzoquinone. The solubility of the substituted benzoquinones can be reduced by substitution of groups such as alkyl or aryl groups onto the ring. Solubility of the compound of formula (I) is an important factor in the proper functioning of the sensor in a flowing stream. Low solubility in water and aqueous phases is helpful in providing stability and conveniently, for use in a flowing stream, the compound of formula (I) should have a solubility of from 400 mg/L to 10,000 mg/L in pure water.

Sensors of the invention for use in static solution will advantageously comprise substituted benzoquinones which have higher solubility compared to unsubstituted benzoquinone. The solubility of the substituted benzoquinones can be increased by substitution of groups such as hydroxy, alkoxy or hydroxyalkyl onto the ring. Relatively high solubility in water and aqueous phases is helpful in providing enhanced sensitivity of the sensor response in static solution. Conveniently in this embodiment, the compound of formula (I) will have a solubility of at least 10,000 mg/L in pure water.

Solubility in common organic solvents is desirable to facilitate fabrication of the sensors of the present invention, and conveniently the compound of formula (I) will have a solubility of at least 20,000 mg/L and preferably higher, in at least one of methanol, ethanol, propanol, other lower alkanols, chloroform, dichloromethane or other chlorinated alkanes and acetone and other low molecular weight solvents.

Preferably the mediator is specific to reduced enzymes, i.e. under the conditions of the analysis, the mediator only accepts electrons from the redox site of a reduced enzyme. In practice it is likely that this will be the case when operating at the preferred potential (see below). However, specificity is not essential and the system may be operated satisfactorily provided that the mediator is selective for reduced enzyme, i.e. under the conditions of the analysis the mediator tends to accept electrons from the redox site of the reduced enzyme in preference to any other electron donor available to the mediator.

In one embodiment of the invention, groups R¹, R², R³ and R⁴ may be selected from any alkyl group, preferably C₁₋₃ alkyl. The alkyl group includes methyl, ethyl and propyl and is preferably methyl. Another suitable configuration has R¹, R² and R³ all hydrogen atoms and group R⁴ a phenyl group. If there is more than one alkyl group or a phenyl group, these substituents render the substituted benzoquinone relatively insoluble which provides a mediator of particular advantage for use in a flowing stream. It is preferred in that embodiment that the substituted benzoquinones used have a solubility of not more than 10,000 mgL⁻¹ in water at room temperature (25°C), and not less than 1000 mgL⁻¹. As specific examples of useful compounds there may be mentioned 2,6-dimethyl-1,4-benzoquinone, which has a solubility of 7888 mgL⁻¹ in water at 25°C, tetramethyl-1,4-benzoquinone, which has a solubility of 443 mgL⁻¹ in water at 25°C, phenyl-1,4-benzoquinone, which has a solubility of 1135 mgL⁻¹ in water at 25°C, and 2,5-dimethyl-1,4-benzoquinone, which has a solubility of 7010 mgL⁻¹ in water at 25°C, which may be compared to the parent compound 1,4-benzoquinone which has a solubility of 11,000 mgL⁻¹ in water at 18°C.

An alkyl group or groups may be substituted by one or more substituents provided that these do not have a detrimental effect on the activity of the mediator compounds.

In another embodiment of the invention, groups R¹, R², R³ or R⁴ may be an alkoxy group or a hydroxyalkyl group, preferably C₁₋₃ alkoxy or hydroxy C₁₋₃ alkyl. The alkoxy group includes methoxy, ethoxy and propoxy and is preferably methoxy. The hydroxyalkyl group includes hydroxymethyl, hydroxyethyl and hydroxypropyl and is preferably hydroxymethyl. If there is more than one alkoxy group or hydroxyalkyl group, these substituents render the substituted benzoquinone relatively soluble which provides mediator of particular advantage for use in static solution. It is preferred that in this aspect of the invention, that the substituted benzoquinones used have a solubility of at least 10,000 mgL⁻¹ in water at room temperature (25°C). As specific examples of useful compounds there may be mentioned 2,6-dimethoxy-1,4-benzoquinone which has a solubility of 70,600 mgL⁻¹ in water at 25°C, 2,3-dimethoxy-5-methyl-1,4-benzoquinone which has a solubility of 11,000 mgL⁻¹ in water at 25°C and 2-hydroxymethyl-6-methoxy-1,4-benzoquinone.

Some of the compounds useful as mediators are known and are commercially available. Alternatively, they may be made by the application or adaptation of known techniques.

The mediator compounds disclosed herein are useful in a variety of amperometric sensor devices and electrode configurations. The sensors may be based on a 2 or 3 electrode system and may be of the disposable (single use) or reusable/semi-disposable type. The sensors may be used either in a flowing stream of solution or in static solution, depending on the relative solubility of the mediator used. In its simplest form the sensor comprises two electrodes (working and counter) which in use are contacted with the sample being analysed. One electrode, the working electrode, is coated with the mediator compound. Mediator may be applied to the electrode by deposition from a solution of the mediator in a readily evaporable organic liquid. For mediator that is fairly soluble in aqueous solution, it may also be applied to the electrode by deposition from an aqueous solution of the mediator. When the sensor is being used to determine the concentration of an analyte such as glucose the mediator is coated with a suitable enzyme. The enzyme can be immobilised on the surface of the mediator by conventional techniques such as by use of a self-sustaining gel layer and/or by use of a retention layer, which is permeable to the analyte. US 4,711,245 describes in greater detail ways in which the mediator and, when used, the enzyme may be fixed on the working electrode.

The electrode substrate is chosen from conventional materials such as carbon pellets, carbon inks, metallized carbon and metals (such as platinum or palladium), carbon rods, pencil leads and carbon rods loaded with metal powder. Conventional electrode configurations which may be used include those disclosed in US 4,711,245, US 5,200,051 and US 5,288,636. The basic chemical and electrochemical transformations associated with the present invention are shown below with reference to the glucose/glucose oxidase system. Prior to introduction of the sample to be analysed a potential of about +200mV (versus Ag/AgCl) is applied to the sensor electrode. This potential is sufficient to cause oxidation of the mediator at the working electrode, i.e. conversion of hydroquinone to the corresponding benzoquinone. When the electrodes are contacted with the sample to be analysed, the enzyme at the working electrode acts on the glucose resulting in the formation of the reduced form of the enzyme. The reaction proceeds as in the reaction scheme below:

GOD_{OX} + glucose → GOD_{RED} + gluconolactone

The reduced form of the enzyme reduces the oxidized form of the mediator as follows:

GOD_{RED} + M_{OX} → GOD_{OX} + M_{RED}

Then, under the applied potential, the reduced form of the mediator at the working electrode is converted to the oxidized form and a diffusion limited current generated. This current can be measured and correlated to the concentration of analyte in the sample.

At the electrode potential involved (+200 mV) there may be some oxidation of interferants. Typically in blood and plasma samples these are ascorbate, urate and paracetamol. A diffusion limiting layer may be applied to the working electrode to extend the sensor to measurement of higher analyte concentrations. This layer may also reduce the diffusion of some interferents to the electrode by acting as a diffusion-limiting barrier. This layer can also act as an electrostatic barrier to diffusion of interferents if it contains negatively charged groups. Examples of materials for use as the diffusion-limiting layer include Nafion^{™} and cellulose acetate. An effective outer membrane results in a sensor response which is an accurate reflection of the reduced enzyme concentration in the sample. The reduced enzyme concentration may be correlated to analyte concentration.

It is envisaged that the sensors of the invention will find most practical utility in the measurement of glucose in blood samples, although they may be also used for other medical and non-medical applications, for example in the food industry.

### Brief description of the accompanying drawings:

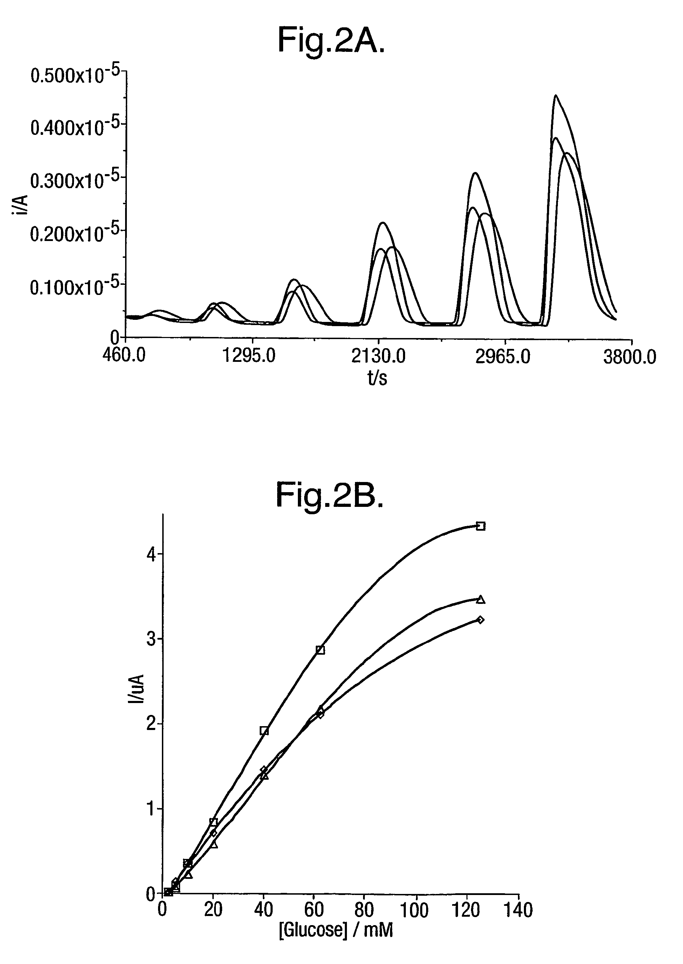

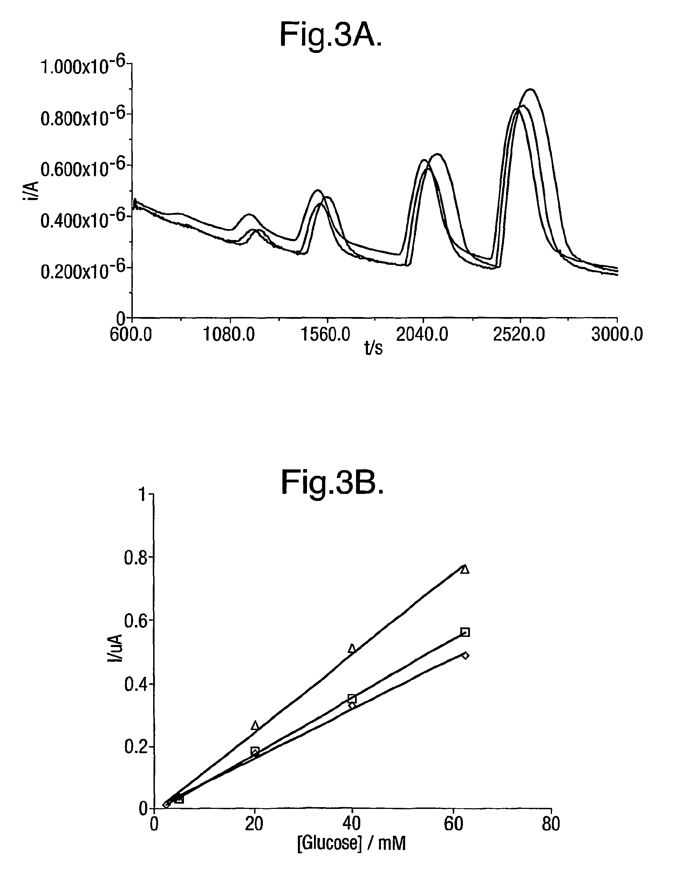

Figure 1:
   A sketch of an embodiment of the amperometric sensor of the invention in the form of a flow cell. The cell consists of a lower part (A) containing the sensors and an upper part (B) containing the inlet and outlet for fluid flow. Each sensor is surrounded by a recess of 0.5 mm width and 0.1 mm depth. The top of the sensor surface is level with the bottom of the recess. The upper part (B) contains a rubber O-ring seal and is clamped in place over the sensors. The parts shown are: sensor 1 (1), sensor 2 (2), Ag/AgCl reference electrode (3), sensor 3 (4), flow inlet (5), flow outlet (6), hole for screw (7), O-ring (8) and 0.2 mm recess over sensors and between inlet and outlet (9).
Figure 2A:
   Real time calibration traces obtained by flow injection from three 2,6-dimethyl-1,4-benzoquinone mediated glucose sensors showing the responses to injections of glucose solutions containing 5, 10, 20, 40, 62.5 and 125 mM glucose. The carrier used was bis-tris saline buffer, pH 6.95.
Figure 2B:
   The calibration plots for the three 2,6-dimethyl-1,4-benzoquinone mediated glucose sensor responses shown in Figure 2B.
Figure 3A:
   Real time calibration traces obtained by flow injection from three phenyl-1,4-benzoquinone mediated glucose sensors showing the responses to injections of glucose solutions containing 2.5, 5, 20, 40 and 62.5 mM glucose. The carrier used was bis-tris saline buffer, pH 6.95.
Figure 3B:
   The calibration plots for the three phenyl-1,4-benzoquinone mediated glucose sensor responses shown in Figure 3B.
Figures 4A and 4B show the current response and the corresponding calibration plot in the response of 2,3-dimethoxy-5-methyl-1,4-benzoquinone/sarcosine oxidase to sarcosine.
Figures 5A and 5B show the current response and the corresponding calibration plot in the response of 2,6-dimethyl-1,4-benzoquinone/sarcosine oxidase to sarcosine.
Figures 6A and 6B show the current response and the corresponding calibration plot in the response of 2,5-dimethyl-1,4-benzoquinone/sarcosine oxidase to sarcosine.
Figure 7A and 7B show the current response and the corresponding calibration plot in the response of 2,5-dimethyl-1,4-benzoquinone/glucose oxidase to glucose.
Figures 8A and 8B show the current response and the corresponding calibration plot in the response of 2,3-dimethoxy-5-methyl-1,4-benzoquinone/glucose oxidase to glucose.
Figures 9A and 9B show the current response and the corresponding calibration plot in the response of 2-hydroxymethyl-6-methoxy-1,4-benzoquinone/glucose oxidase to glucose.

### EXAMPLES

### Example 1 Electrode construction

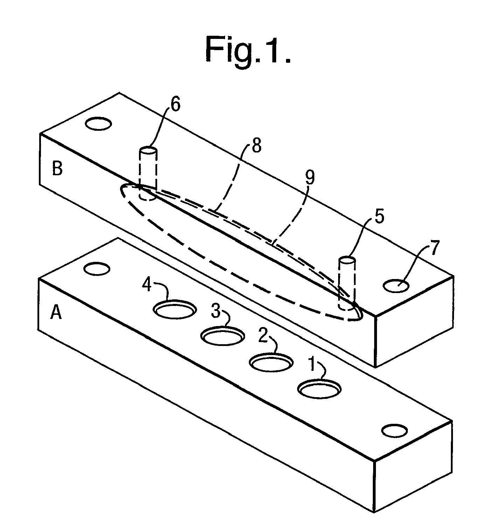

The amperometric flow cell was supplied by Drew Scientific, Cumbria, U.K. Figure 1 shows a sketch of the flow cell, including the sensors. The cell was made of either PVC or nylon. Carbon pellets (2mm diameter and 4mm length) were press fitted into the holes in the lower part (A) so that the front face was flush with the bottom of the recess. Electrical contact was made to the back face of the pellets. One of the carbon pellets was modified with Ag/AgCl paste to act as both a pseudo-reference electrode and counter electrode.

### Example 2 2,6-dimethyl-1,4-benzoquinone mediated glucose sensor

Unmodified carbon pellets, inserted into the bottom part of the flow cell as described in Example 1, were prepared as glucose sensors by successively depositing a mediator layer, an enzyme layer and an outer diffusion limitation layer. 2 - 4µL of 0.5 M 2,6-dimethyl-1,4-benzoquinone solution in acetone was deposited into the recess surrounding each pellet and allowed to dry for 3 minutes. A solution containing a mixture of glucose oxidase GOX (2 - 4 U/µL), PVA (0.09%) and Nafion (0.31 %) was prepared and a total of 4 µL of the solution was deposited on top of the benzoquinone layer of each sensor to form the enzyme sensing layer. The sensors were air dried for 20 minutes before a total of 3 µL of 2.5% Nafion solution was deposited onto each sensor. The sensors were air dried for at least 4hr before use.

### Example 3 Use of 2,6-dimethyl-1,4-benzoquinone glucose sensor

Flow injection chronoamperometry was used to show the activity of these sensors to glucose. A two electrode system was used, with the three sensors of Example 2 as the working electrodes, and the pellet modified with silver/silver chloride acting as the reference and counter electrode. An AutoLab (Eco Chemie B.V.) electrochemical system was used for the measurements. The carrier buffer was pH 6.95 bis-tris buffer (40 mM), containing 142 mM NaCl, 0.8 mM Na₂EDTA and 4.2 mM KCl. The working electrodes were poised at a potential of +200 mV versus the silver/silver chloride reference electrode. Figure 2A shows anodic current peaks corresponding to injections of glucose. Calibration plots resulting from the amperometry data in Figure 2A are shown in Figure 2B. The linear range of response is from 0 to 60 mM glucose.

### Example 4 Phenyl-1,4-benzoquinone mediated glucose sensor

Unmodified carbon pellets, inserted into the bottom part of the flow cell as described in Example 1, were prepared as glucose sensors in an identical manner to the sensors in Example 2, with the exception of the use of 0.5 M phenyl-1,4-benzoquinone solution in place of 2,6-dimethyl-1,4-benzoquinone solution.

### Example 5 Use of phenyl-1,4-benzoquinone glucose sensor

Flow injection chronoamperometry was used to demonstrate the response of the sensors in Example 4 to glucose, using the same experimental methodology given in Example 3. Figure 3A shows anodic current peaks corresponding to injections of glucose. Calibration plots resulting from the amperometry data in Figure 3A are shown in Figure 3B. The linear range of response is from 0 to 60 mM glucose.

### Example 6

Chronoamperometry was used to show the mediator ability of some compounds in solution to sarcosine oxidase. The change in current at a glassy carbon electrode was observed on consecutive addition of aliquots of 1 M sarcosine stock solution to buffer (pH 7.4) containing 100 units/ml sarcosine oxidase and either 0.2 M 2,6-dimethyl-1,4-benzoquinone, 0.2 M 2,5-dimethyl-1,4-benzoquinone or a saturated solution of 2,3-dimethoxy-5-methyl-1,4-benzoquinone.

The electrode potential was held at + 250 mV vs. Ag/AgCl/sat/KCI and platinum counter electrode was used.

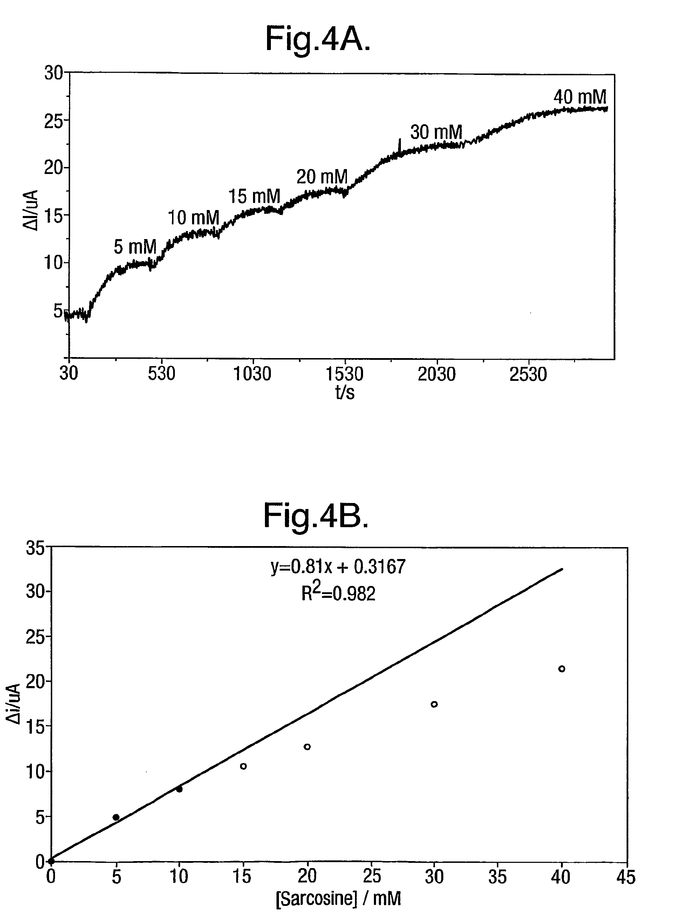

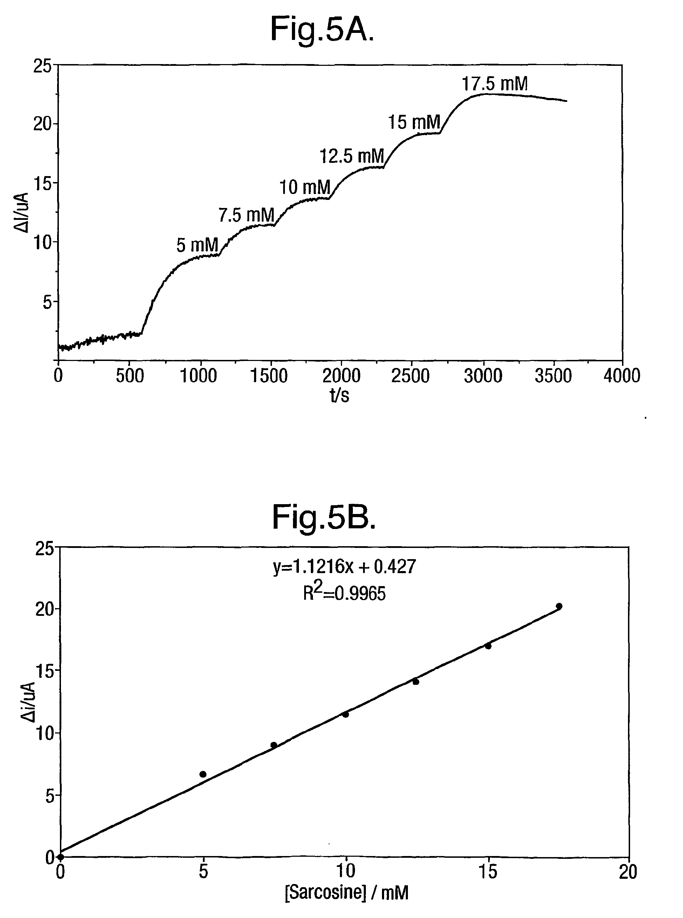

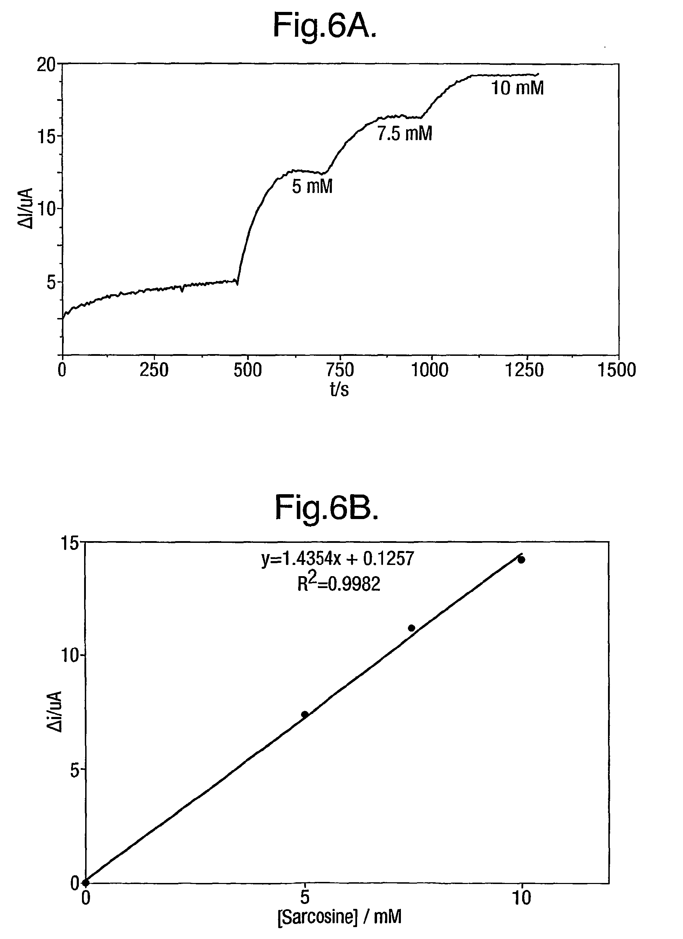

Figure 4A shows the current response on the addition of sarcosine to a solution containing 2,3-dimethoxy-5-methyl-1,4-benzoquinone and sarcosine oxidase. Figure 4B shows the corresponding calibration plot. Figure 5A shows the current response on the addition of sarcosine to a solution containing 2,6-dimethyl-1,4-benzoquinone and sarcosine oxidase, and Figure 5B shows the corresponding calibration plot. Figure 6A shows the current response of 2,5-dimethyl-1,4-benzoquinone/sarcosine oxidase to sarcosine, and Figure 6B shows the corresponding calibration plot.

### Example 7

Chronoamperometry was used to show the mediator ability of some compounds in solution to glucose oxidase. The change in current at a glassy carbon electrode was observed on consecutive addition of aliquots of 1 M glucose stock solution to buffer (pH 7.4) containing 100 units/ml glucose oxidase and either 0.2 M 2,5-dimethyl-1,4-benzoquinone, a saturated solution of 2,3-dimethoxy-5-methyl-1,4-benzoquinone or a saturated solution of 2-hydroxymethyl-6-methoxy-1,4-benzoquinone.

The electrode potential was held at + 250 mV vs. Ag/AgCl/sat/KCl and platinum counter electrode was used.

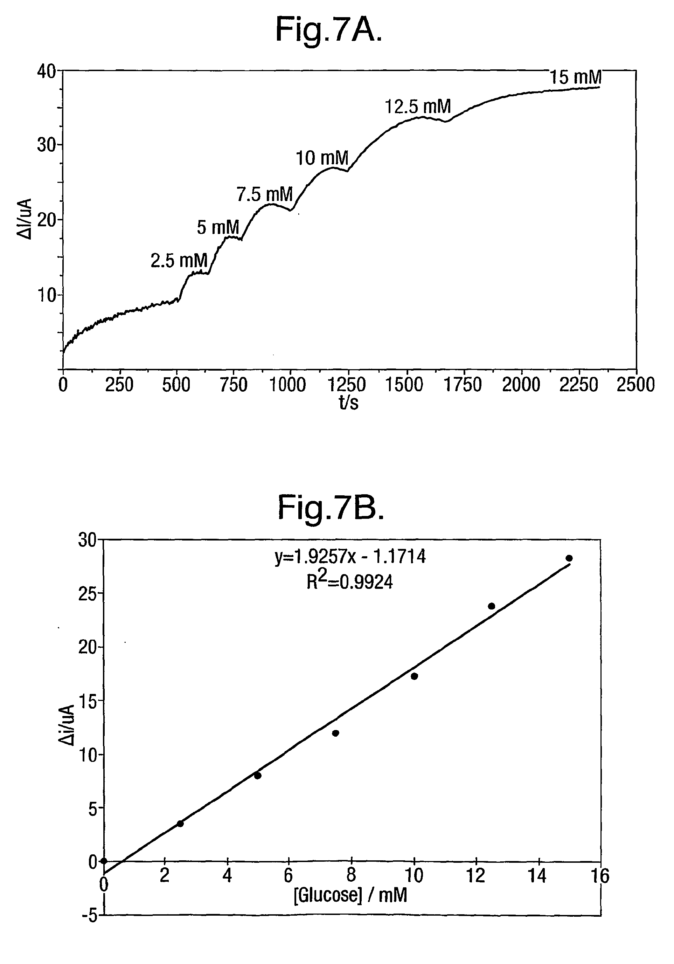

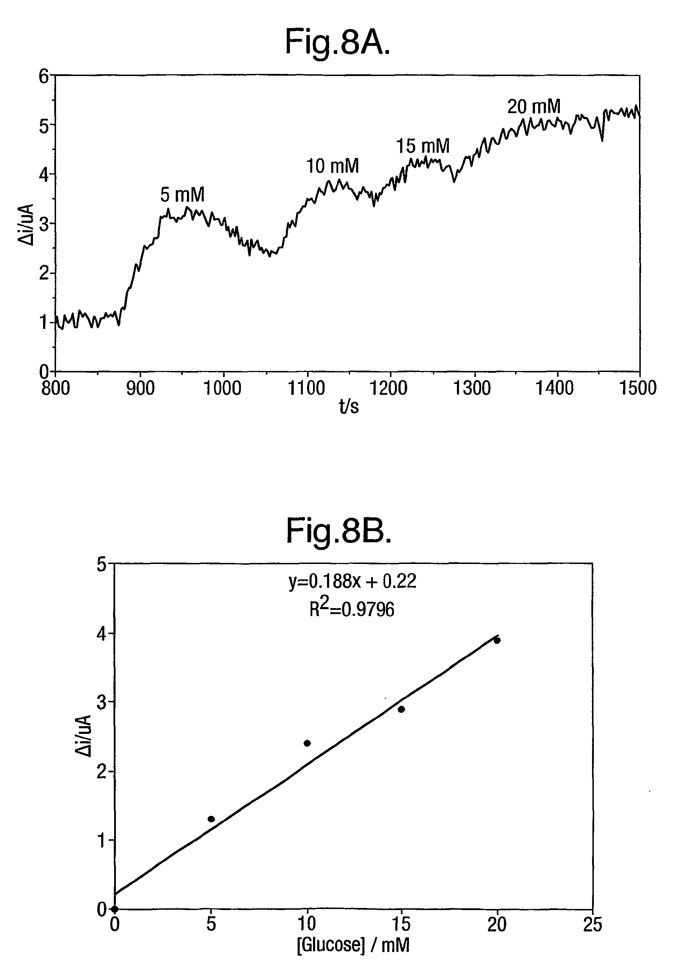

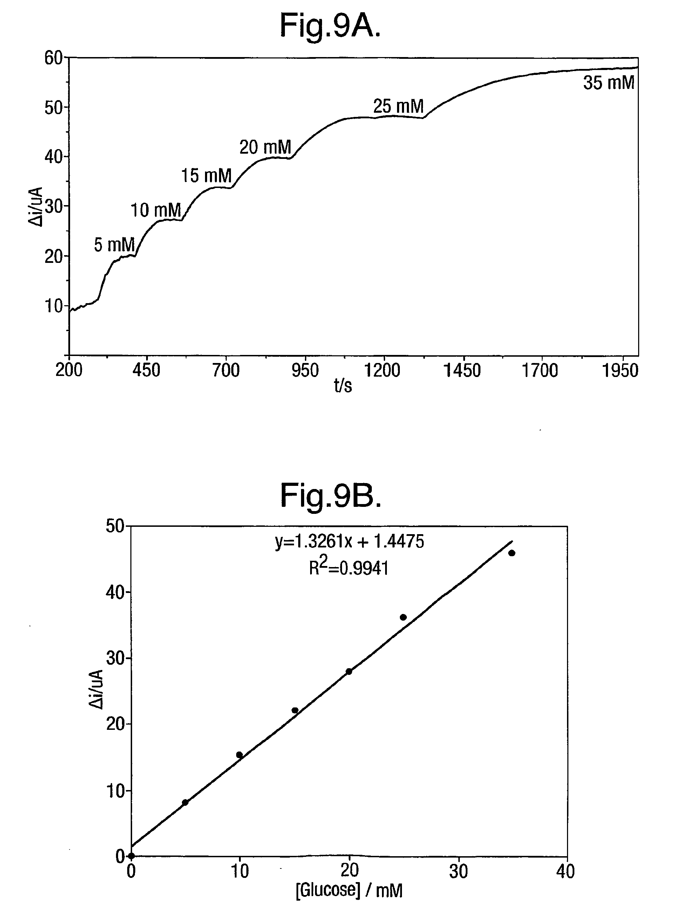

Figure 7A shows the current response of 2,5-dimethyl-1,4-benzoquinone/glucose oxidase to glucose, and Figure 7B shows the corresponding calibration plot. Figure 8A shows the current response of 2,3-dimethoxy-5-methyl-1,4-benzoquinone/glucose oxidase to glucose and Figure 8B shows the corresponding calibration plot. Figure 9A shows the current response of 2-hydroxymethyl-6-methoxy-1,4-benzoquinone/glucose oxidase to glucose, and Figure 9B shows the corresponding calibration plot.

## Claims

1. An amperometric sensor suitable for determining the concentration of analyte in a sample, said sensor comprising an oxidase enzyme and a substituted 1,4-benzoquinone compound which, in oxidized form, functions as a mediator selective for reduced enzyme and has an oxidation potential lower than that of unsubstituted 1,4-benzoquinone.

2. A sensor according to claim 1, wherein the analyte is glucose and the enzyme is glucose oxidase.

3. A sensor according to claim 1, wherein the substituted 1,4-benzoquinone compound has a solubility in pure water that is reduced compared to that of unsubstituted 1,4-benzoquinone.

4. A sensor according to claim 3, wherein the solubility of the substituted 1,4-benzoquinone compound in pure water is from 400 to 10,000 mg/l.

5. A sensor according to claims 1 to 4, wherein the substituted 1,4-benzoquinone compound is of formula (I): 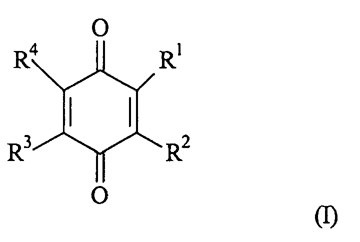 wherein R¹, R², R³ and R⁴ are the same or different and at least one of R¹, R², R³ and R⁴ is an alkyl group or a phenyl group.

6. A sensor according to claim 5, wherein the alkyl groups have from 1 to 3 carbon atoms.

7. A sensor according to claim 6, wherein the 1,4-benzoquinone compound is chosen from 2,6-dimethyl-1,4-benzoquinone, tetramethyl-1,4-benzoquinone, methyl-1,4-benzoquinone, 2,5-dimethyl-1,4-benzoquinone and phenyl-1,4-benzoquinone.

8. A sensor according to claim 1, wherein the substituted 1,4-benzoquinone compound has a solubility in pure water that is increased compared to that of unsubstituted 1,4-benzoquinone.

9. A sensor according to claim 8, wherein the solubility of the substituted 1,4-benzoquinone compound in pure water is at least 10,000 mg/l.

10. A sensor according to claims 1, 2, 8 or 9, wherein the 1,4-benzoquinone compound is of formula (I) wherein R¹, R², R³ and R⁴ are the same or different and at least one of R¹, R², R³ and R⁴ is an alkoxyl group or a hydroxyalkyl group.

11. A sensor according to claim 10, wherein the alkoxyl group or hydroxyalkyl group have from 1 to 3 carbon atoms.

12. A sensor according to claim 10 or 11, wherein the benzoquinone compound is chosen from 2,6-dimethoxy-1,4-benzoquinone, 2,3-dimethoxy-5-methyl-1,4-benzoquinone and 2-hydroxymethyl-6-methoxy-1,4-benzoquinone.

13. A cartridge for an amperometric sensor suitable for measuring analyte in a sample, which cartridge comprises an enzyme and a substituted benzoquinone compound as defined in any one of the claims 1 to 12.

14. Use of an amperometric sensor as claimed in any one of claims 1 to 12, for determining the concentration of an analyte in a sample, wherein the enzyme of the sensor reacts with the analyte to produce reduced enzyme which is then regenerated by the mediator.

## Patentansprüche

1. Amperometrischer Sensor, der zum Ermitteln der Konzentration von Analyt in einer Probe geeignet ist, wobei der Sensor ein Oxidaseenzym und eine substituierte 1,4-Benzochinon-Verbindung umfasst, die in oxidierter Form als Mediator selektiv für reduziertes Enzym dient und ein niedrigeres Oxidationspotenzial als nicht substituiertes 1-4-Benzochinon aufweist.

2. Sensor nach Anspruch 1, **dadurch gekennzeichnet, dass** das Analyt Glucose ist und das Enzym Glucose-Oxidase ist.

3. Sensor nach Anspruch 1, **dadurch gekennzeichnet, dass** die substituierte 1,4-Benzochinon-Verbindung eine Löslichkeit in reinem Wasser aufweist, die verglichen mit der von nicht substituiertem 1,4-Benzochinon reduziert ist.

4. Sensor nach Anspruch 3, **dadurch gekennzeichnet, dass** die Löslichkeit der substituierten 1,4-Benzochinon-Verbindung in reinem Wasser von 400 bis 10.000 mg/l reicht.

5. Sensor nach den Ansprüchen 1 bis 4, **dadurch gekennzeichnet, dass** die 1,4-Benzochinon-Verbindung die Formel (I) aufweist: 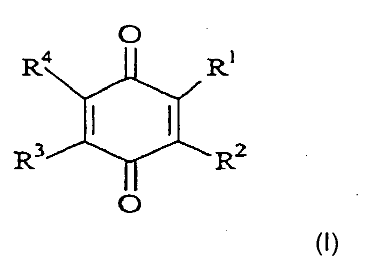 wobei R¹, R², R³ und R⁴ gleich oder unterschiedlich sind und mindestens eines von R¹, R², R³ und R⁴ eine Alkylgruppe oder eine Phenylgruppe ist.

6. Sensor nach Anspruch 5, **dadurch gekennzeichnet, dass** die Alkylgruppen 1 bis 3 Kohlenstoffatome aufweisen.

7. Sensor nach Anspruch 6, **dadurch gekennzeichnet, dass** die 1,4-Benzochinon-Verbindung aus 2,6-Dimethyl-1,4-benzochinon, Tetramethyl-1,4-benzochinon, Methyl-1,4-benzochinon, 2,5-Dimethyl-1,4-benzochinon und Phenyl-1,4-benzochinon gewählt wird.

8. Sensor nach Anspruch 1, **dadurch gekennzeichnet, dass** die substituierte 1,4-Benzochinon-Verbindung eine Löslichkeit in reinem Wasser aufweist, die verglichen mit der von nicht substituiertem 1,4-Benzochinon erhöht ist.

9. Sensor nach Anspruch 8, **dadurch gekennzeichnet, dass** die Löslichkeit der substituierten 1,4-Benzochinon-Verbindung in reinem Wasser mindestens 10.000 mg/l beträgt.

10. Sensor nach den Ansprüchen 1, 2, 8 oder 9, **dadurch gekennzeichnet, dass** die 1,4-Benzochinon-Verbindung die Formel (I) aufweist, wobei R¹, R², R³ und R⁴ gleich oder unterschiedlich sind und mindestens eines von R¹, R², R³ und R⁴ eine Alkoxylgruppe oder eine Hydroxyalkylgruppe ist.

11. Sensor nach Anspruch 10, **dadurch gekennzeichnet, dass** die Alkoxylgruppe oder Hydroxyalkylgruppe 1 bis 3 Kohlenstoffatome aufweist.

12. Sensor nach Anspruch 10 oder 11, **dadurch gekennzeichnet, dass** die Benzochinon-Verbindung aus 2,6-Dimethoxy-1,4-benzochinon, 2,3-Dimethoxy-5-methyl-1,4-benzochinon und 2-Hydroxmethyl-6-methoxy-1,4-benzochinon gewählt wird.

13. Patrone für einen amperometrischen Sensor, der zum Messen von Analyt in einer Probe geeignet ist, wobei die Patrone ein Enzym und eine substituierte Benzochinon-Verbindung nach einem der Ansprüche 1 bis 12 umfasst.

14. Verwendung eines amperometrischen Sensors nach einem der Ansprüche 1 bis 12 zum Ermitteln der Konzentration eines Analyts in einer Probe, wobei das Enzym des Sensors mit dem Analyt reagiert, um ein reduziertes Enzym zu erzeugen, das dann durch den Mediator regeneriert wird.

## Revendications

1. Capteur ampérométrique employé pour déterminer la concentration d'un analyte dans un échantillon, ledit capteur comprenant une enzyme oxydase et un composé 1,4-benzoquinone substitué qui, lorsqu'il est sous forme oxydée, fonctionne comme un médiateur sélectif d'une enzyme réduite, et a un potentiel d'oxydation inférieur à celui de la 1,4-benzoquinone non substituée.

2. Capteur selon la revendication 1, dans lequel l'analyte est le glucose et l'enzyme est la glucose oxydase.

3. Capteur selon la revendication 1, dans lequel le composé 1,4-benzoquinone substitué a une solubilité dans l'eau pure qui est réduite en comparaison avec celle de la 1,4-benzoquinone non substituée.

4. Capteur selon la revendication 3, dans lequel la solubilité du composé 1,4-benzoquinone substitué dans l'eau pure est de 400 à 10 000 mg/L.

5. Capteur selon les revendications 1 à 4, dans lequel le composé 1,4-benzoquinone substitué a pour formule (I) : 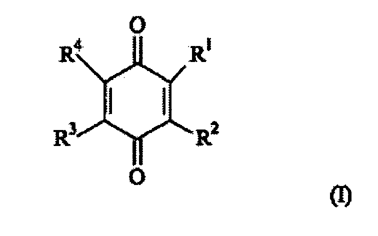 où R¹, R², R³ et R⁴ sont identiques ou différents et au moins l'un de R¹, R², R³ et R⁴ est un groupe alkyle ou un groupe phényle.

6. Capteur selon la revendication 5, dans lequel les groupes alkyle comportent 1 à 3 atomes de carbone.

7. Capteur selon la revendication 6, dans lequel le composé 1,4-benzoquinone est choisi parmi la 2,6-diméthyl-1,4-benzoquinone, la tétraméthyl-1,4-benzoquinone, la méthyl-1,4-benzoquinone, la 2,5-diméthyl-1,4-benzoquinone et la phényl-1,4-benzoquinone.

8. Capteur selon la revendication 1, dans lequel le composé 1,4-benzoquinone substitué a une solubilité dans l'eau pure qui est augmentée en comparaison avec celle de la 1,4-benzoquinone non substituée.

9. Capteur selon la revendication 8, dans lequel la solubilité du composé 1,4-benzoquinone substitué dans l'eau pure est d'au moins 10 000 mg/L.

10. Capteur selon les revendications 1, 2, 8 ou 9, dans lequel le composé 1,4-benzoquinone a pour formule (I) où R¹, R², R³ et R⁴ sont identiques ou différents et au moins l'un de R¹, R², R³ et R⁴ est un groupe alcoxyle ou hydroxyalkyle.

11. Capteur selon la revendication 10, dans lequel le groupe alcoxyle ou hydroxyalkyle comporte 1 à 3 atomes de carbone.

12. Capteur selon la revendication 10 ou 11, dans lequel le composé benzoquinone est choisi parmi la 2,6-diméthoxy-1,4-benzoquinone, la 2,3-diméthoxy-5-méthyl-1,4-benzoquinone et la 2-hydroxyméthyl-6-méthoxy-1,4-benzoquinone.

13. Cartouche pour un capteur ampérométrique approprié pour mesurer un analyte dans un échantillon, laquelle cartouche comprend une enzyme et un composé benzoquinone substitué tel que défini dans l'une quelconque des revendications 1 à 12.

14. Utilisation d'un capteur ampérométrique tel que revendiqué dans l'une quelconque des revendications 1 à 12, pour déterminer la concentration d'un analyte dans un échantillon, où l'enzyme du capteur réagit avec l'analyte pour produire une enzyme réduite qui est ensuite régénérée par le médiateur.
